(19) Europäisches Patentamt
European Patent Office
Office européen des brevets

(11) Numéro de publication : **0 349 382 B1**

# (12) FASCICULE DE BREVET EUROPEEN

(45) Date de publication du fascicule du brevet : **14.10.92 Bulletin 92/42**

(51) Int. Cl.$^5$ : **C07C 37/86,** C07C 39/15

(21) Numéro de dépôt : **89401749.0**

(22) Date de dépôt : **21.06.89**

## (54) Procédé de purification de dihydroxybiphényles.

(30) Priorité : **29.06.88 FR 8808725**

(43) Date de publication de la demande : **03.01.90 Bulletin 90/01**

(45) Mention de la délivrance du brevet : **14.10.92 Bulletin 92/42**

(84) Etats contractants désignés : **AT BE CH DE ES FR GB GR IT LI LU NL SE**

(56) Documents cités :
**FR-A- 493 452**
**FR-E- 51 712**
**US-A- 2 208 808**

(73) Titulaire : **RHONE-POULENC CHIMIE**
**25, quai Paul Doumer**
**F-92408 Courbevoie Cédex (FR)**

(72) Inventeur : **Nonn, Alain**
**1, rue du Rhin**
**F-68120 Pfastatt (FR)**

(74) Mandataire : **Vignally, Noel et al**
**Rhône-Poulenc Chimie Service Brevets**
**Chimie 25 Quai Paul Doumer**
**F-92408 Courbevoie Cédex (FR)**

EP 0 349 382 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de purification de dihydroxybiphényles. Elle concerne plus particulièrement la purification du dihydroxy -4,4′ biphényle.

Le dihydroxy -4,4′ biphényle est préparé par exemple selon le brevet américain US 4475000 par hydrolyse de dibromo -4,4′ biphényle en présence d'un catalyseur au cuivre à une température supérieure à 300°C. Lors de la préparation du dérivé dibromé en 4,4′ il est impossible d'éviter la présence de dérivés monobromés ou dibromés en d'autres positions que la position 4,4′.

Lors de l'hydrolyse de ce mélange complexe ou même du dérivé dibromé pur il y a toujours formation de monohydroxybiphényle et de faibles quantités de produits plus lourds de polycondensation contenant au moins deux motifs biphényles. Il est difficile de séparer entre eux ces différents composés ayant des structures très voisines et donc des propriétés physiques peu différenciées.

Le dihydroxy -4,4′ biphényle peut aussi être préparé selon le brevet américain US 4447656 par couplage oxydant du ditertiobutylphénol puis réduction et déalkylation. Au cours de cette réaction sont formés d'autres isomères que le tétratertiobutyl -2,2′,6,6′ p.p′ biphénol.

Au cours de ces réactions successives apparaissent comme dans le brevet précédemment cité des réactions incomplètes avec formation de monohydroxybiphényles, des réactions secondaires avec formation de dérivés lourds polycondensés tels que par exemple les tétrahydroxytétraphenyles ou les bis (hydroxybiphényles) éthers.

Le dihydroxy -4,4′ biphényle est une matière première utilisée dans la synthèse de polymères de haute technicité où il est nécessaire d'avoir des chaînes polymériques, exemptes de ramification. Il est donc indispensable que le dihydroxy -4,4′ biphényle soit pur sans isomères.

La présente invention permet à partir des solutions issues de l'hydrolyse des dibromobiphényles ou de la déalkylation des tétratertiobutylbiphénols d'obtenir une solution pure de dihydroxybiphényle.

La présente invention a pour objet un procédé de purification du dihydroxy -4,4′ biphényle caractérisé en ce qu'on effectue une estérification du mélange réactionnel issu de la préparation du biphényle, avec un dérivé de l'acide acétique en présence d'un acide et qu'on laisse cristalliser le diester de biphényle.

Ce procédé particulièrement simple et économique permet d'obtenir à partir de solutions contenant environ 15 % en poids de produits secondaires un biphényle présentant une pureté de l'ordre de 99 %.

On entend par derivé d'acide les halogénures d'acides et de préférence le chlorure d'acide ainsi que les anhydrides d'acides ou les esters.

Parmi l'ensemble de ces dérivés on préfère utiliser le chlorure d'acétyle ou l'anhydride acétique.

L'estérification est réalisée en présence d'un catalyseur d'estérification qui peut être

un acide organique tel que notamment :

- l'acide benzène sulfonique
- l'acide méthane sulfonique
- l'acide trifluoroacétique
- l'acide trifluorométhane sulfonique

un acide minéral tel que notamment :

- l'acide sulfurique
- l'acide chlorhydrique

une résine acide telle que notamment :

- une résine benzène sulfonique

L'estérification peut avoir lieu en présence ou en l'absence de solvant.

Lorsque l'on utilise un solvant celui-ci peut correspondre à l'acide formé lors de l'estérification ou à un solvant choisi notamment parmi les halogéno alcanes, tel que le dichloroéthane, parmi les solvants aromatiques tel que notamment le toluène, parmi les esters tel que l'acétate d'éthyle, parmi les cétones tel que la méthylisobutylcétone, parmi les alcools tel que le méthanol. Les solvants précédemment cités le sont à titre illustratif et non limitatif. On préfère réaliser l'esterification au sein de l'acide acétique.

En ce qui concerne les conditions réactionnelles on réalise l'estérification de préférence à une température supérieure ou égale à 80°C et tout particulièrement à la température de reflux du milieu réactionnel.

La réaction d'estérification est effectuée sous pression supérieure ou égale à la pression atmosphérique.

La cristallisation est effectuée selon toute technique connue par l'homme de l'art soit par refroidissement du milieu réactionnel, soit par évaporation du milieu sous pression atmosphérique ou sous pression réduite.

L'invention sera plus complètement décrite à l'aide des exemples suivants qui ne doivent pas être considérés comme limitatifs de l'invention.

EXEMPLE 1

A partir d'un mélange contenant :

87 % de dihydroxy-4,4' biphényle

2 % d'hydroxy-4 biphényle

11 % de composés de formules

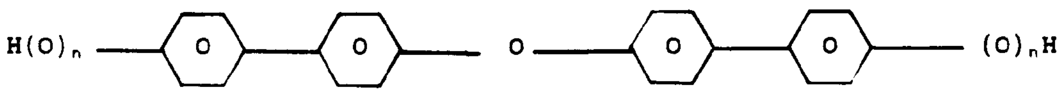

et

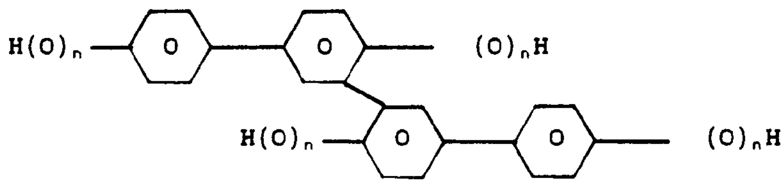

avec n = 0 ou 1

32,75 g du mélange ci-dessus sont estérifiés de la façon suivante :

- mise en suspension dans 150 ml d'acide acétique ;
- addition de 36,5 g d'anhydride acétique et de 0,28 g d'acide paratoluènesulfonique ;
- chauffage à reflux pendant trois heures avec agitation magnétique ;
- refroidissement lent sous agitation magnétique : il a formation de cristaux blancs ;
- après filtration, les cristaux sont lavés à l'eau puis séchés sous vide à 50°C.

Le rendement est de 90 %.

Le dosage par chromatographie liquide haute performance donne une pureté supérieure à 99,5 %.

Point de fusion : 160°C

| Microanalyse : | | théorique | trouvé |
|---|---|---|---|
| | C : | 71,10 | 71,15 |
| | H : | 5,22 | 5,17 |

EXEMPLE 2

A partir d'un mélange contenant :

84,9 % de dihydroxy-4,4' biphényle

15,1 % de dihydroxy-2,2' biphényle

5,58 g du mélange ci-dessus sont estérifiés selon la même méthode que pour l'exemple 1.

Le rendement en diacétoxy-4,4' biphényle est de 90 %. La pureté est supérieure à 99,5 %

Point de fusion : 160°C

EXEMPLE 3

10,7 g du mélange utilisé dans l'exemple 1 sont estérifiés dans les conditions suivantes :

- dilution dans 60 ml de méthylisobutylcétone ;
- addition de 14,0 g d'anhydride acétique et de 0,11 g d'acide paratoluène sulfonique ;
- chauffage à 100° C pendant 3 heures ;
- refroidissement ;
- filtration des cristaux.

Le rendement en cristaux : 79%

| | |
|---|---|
| Pureté | : 98 %<br>0,1 % d'acétoxy-4 biphényle<br>0,1 % d'acétoxy-4 hydroxy-4' biphénile. |

## Revendications

1. Procédé de purification du dihydroxy -4,4' biphényle caractérisé en ce que l'on effectue une estérification du mélange réactionnel issu de la préparation du biphényle, avec un dérivé de l'acide acétique en présence d'un acide fort et en ce qu'on laisse cristalliser le diester de biphényle.

2. Procédé selon la revendication 1 caractérisé en ce qu'un des dérivés de l'acide acétique est l'anhydride d'acide acétique.

3. Procédé selon la revendication 1 caractérisé en ce que l'acide fort est choisi parmi l'acide sulfurique, l'acide chlorhydrique, l'acide benzènesulfonique, l'acide paratoluèné sulfonique, l'acide trifluorométhane sulfonique, une résine benzène sulfonique

4. Procédé selon la revendication 3 caractérisé en ce que l'acide est l'acide paratoluène sulfonique.

## Patentansprüche

1. Verfahren zur Reinigung von 4,4'-Dihydroxy-biphenyl, dadurch gekennzeichnet, daß man eine Veresterung des bei der Herstellung vom Biphenyl erhaltenen Reaktionsgemisches mit einem Essigsäurederivat in Gegenwart einer starken Säure ausführt und den Diester des Biphenyls auskristallisieren läßt.

2. Verfahren nach Anspruch 1, dadurch gekennzeichnet, daß das Essigsäurederivat Essigsäureanhydrid ist.

3. Verfahren nach Anspruch 1, dadurch gekennzeichnet, daß die starke Säure aus Schwefelsäure, Salzsäure, Benzolsulfonsäure, p-Toluolsulfonsäure, Trifluormethansulfonsäure oder einem Benzolsulfonsäureharz ausgewählt wird.

4. Verfahren nach Anspruch 3, dadurch gekennzeichnet, daß die Säure p-Toluolsulfonsäure ist.

## Claims

1. Process for the purification of 4,4'-dihydroxydiphenyl, characterized in that the reaction mixture from the preparation of the diphenyl is esterified with an acetic acid derivative in the presence of a strong acid and the diphenyl diester is left to crystallize.

2. Process according to claim 1, characterized in that one of the acetic acid derivatives is acetic anhydride.

3. Process according to claim 1, characterized in that the strong acid is chosen from among sulphuric acid, hydrochloric acid, benzene sulphonic acid, paratoluene sulphonic acid, trifluoromethane sulphonic acid and a benzene sulphonic resin.

4. Process according to claim 3, characterized in that the acid is a paratoluene sulphonic acid.